# DEMANDE DE BREVET EUROPEEN

(11) **EP 1 731 911 A1**
(43) Date de publication de la demande: **13.12.2006**
(21) Numéro de dépôt: 05300463.6
(22) Date de dépôt: 07.06.2005
(51) Int. Cl.: G01N 33/68, G01N 33/58

(54) **Procédé utile pour la détection d'encéphalopathies**

(71) Demandeur: INSTITUT NATIONAL DE LA SANTE ET DE LA RECHERCHE MEDICALE (INSERM), 75013 Paris (FR)
(72) Inventeur: Verdier, Jean-Michel, 34980 Saint-Gély-du-Fesc (FR); Gregoire, Catherine, 13127 Vitrolles (FR); Perrier-Rondard, Véronique, 34830 Jacou (FR)
(74) Mandataire: Le Coupanec, Pascale A.M.P.

(57) **Abrégé**

La présente invention concerne un procédé utile pour la détection d'une ESST ou maladie à prions. Elle vise en outre un procédé utile pour l'amplification de l'oligomérisation des isoformes PrP^{Sc} du prion cellulaire.

## Description

La présente invention vise à titre principal un procédé utile pour l'amplification de l'oligomérisation et/ou du titre infectieux des isoformes PrP^{Sc} de la protéine du prion cellulaire PrP^{c}. Elle vise également l'utilisation d'un tel procédé pour le diagnostic, la caractérisation, le suivi clinique, etc., d'encéphalopathie, en particulier d'encéphalopathies subaiguës spongiformes transmissibles (ESST) incluant les encéphalopathies spongiformes bovines (BSE)

Les maladies à prions ou encéphalopathies spongiformes subaiguës transmissibles (ESST) sont des affections neurodégénératives mortelles qui affectent aussi bien les hommes que les animaux. Les ESST comprennent essentiellement la maladie de Creutzfeldt-Jakob, chez l'homme (MCJ ou CJD pour *Creutzfeldt-Jakob disease*), la tremblante chez le mouton et la chèvre et l'encéphalopathie spongiforme bovine (ESB ou BSE pour *bovine spongiform encephalopathy*) chez les bovins. D'autres encéphalopathies ont été mises en évidence chez les félidés, chez le vison ou certains animaux sauvages, tels que le cerf ou l'élan.

L'agent infectieux à l'origine de ces maladies est non conventionnel. Actuellement, l'hypothèse qui domine est celle des "prions", selon laquelle la protéine du prion cellulaire, PrP^{C}, synthétisée par l'organisme hôte serait capable d'adopter une conformation pathologique, appelée PrP^{Sc} qui désigne l'isoforme "scrapie".

Ces deux isoformes présentent la même séquence en acides aminés mais diffèrent dans leur structure secondaire : la PrP^{Sc} présente un contenu significativement plus élevé en feuillets beta plissés, alors que la PrP normale (PrP^{C}) présente un plus grand nombre d'hélices alpha.

Deux propriétés biochimiques permettent, généralement, de distinguer ces deux isoformes :
- la PrP^{Sc} est partiellement résistante aux protéases, notamment à la protéinase K (PK), qui entraîne un clivage de son extrémité N-terminale. Après action de la PK, la PrP^{Sc} est souvent appelée PrP27-30 à cause du poids moléculaire apparent de la forme biglycosylée ; il est généralement admis que le site de clivage de la PrP^{Sc} se situe entre les acides aminés 89 et 90 (Prusiner S.B. et al., Cell, (1984), 38, 127-134) pour les souches habituelles,
- la PrP^{Sc} est insoluble dans les détergents non-ioniques, tels que le Triton X100 ou le Triton 114.

La forme normale de la protéine du prion (PrP^{C}) est en principe complètement dégradée par les protéases et est parfaitement soluble en présence de détergents non-ioniques.

L'accumulation de la PrP^{Sc} dans le cerveau des personnes ou des animaux atteints d'ESST provoque sa dégénérescence. Les recherches sur ces maladies ont pris un essor remarquable depuis la crise de la vache folle en Europe et l'apparition du nouveau variant de la maladie de Creutzfeldt-Jacob chez l'homme (vMCJ). Des progrès conséquents ont été réalisés dans la connaissance de ces agents infectieux non conventionnels. Toutefois, il n'existe pas à l'heure actuelle de traitement disponible pour soigner les patients atteints de MCJ, ni de test de diagnostic précoce.

Durant la dernière décennie, plusieurs molécules présentant une activité "anti-prion" et une structure chimique novatrice ont été identifiées telles que (i) des dendrimères (Supattapone S. et al., Proc. Natl. Acad. Sci. U.S.A. (1999) 96, 14529-34) ; (ii) des dérivés de porphyrines et phtalocyanines (Caughey W.S. et al., Proc. Natl. Acad. Sci. U.S.A. (1998) 21, 12117-22) ; (iii) des dérivés de la quinacrine et de la chlorpromazine (Doh-Ura. et al., J Virol (2000) 74, 4894-7), (Korth C et al., Proc. Natl. Acad. Sci. U.S.A. (2001) 98, 9836-41). Ces drogues sont capables d'éliminer partiellement ou complètement l'infectivité des neuroblastomes de souris ScN2a chroniquement infectées par les prions. Toutefois, leur action est très limitée *in vivo* (Demaimay R et al., J Gen. Virol (1994) 75, 2499-503 ; Priola SA et al., Science (2000) 287, 1503-6). Les raisons permettant d'expliquer le défaut d'efficacité de ces produits *in vivo* sont probablement leur manque de spécificité vis-à-vis de l'agent infectieux, puisque le mécanisme d'action de ces drogues est peu ou pas connu, et la difficulté de ces produits à traverser la barrière hémato-méningée.

Les études de criblage de drogues réalisées pour identifier des produits "anti-prions", ont également conduit à l'identification de deux composés à savoir les 2-amino-6[(2-aminophényl)thio]-4-(2-furyl)pyridine-3,5-dicarbonitrile et N'1-({5-[(4,5-dichloro-1H-imidazol-1-yl)méthyl]2-furyl}carbonyl)-4-méthoxy-benzène-1-sulfonohydrazide, exhibant une activité inhibitrice en culture cellulaire (Perrier V. et al., Proc. Natl. Acad. Sc. U.S.A. (2000) 97, 6073-6078). Cependant, les valeurs d'IC₅₀ (concentration pour laquelle 50 % du taux de PrP^{Sc} dans les cellules est inhibé) obtenues pour ces produits, de l'ordre de 15 µM, ne s'avèrent pas compatibles avec une application *in vivo.*

En terme de diagnostic, les méthodes les plus récentes sont fondées sur une détection sélective de la PrP anormale (PrP^{sc}), liée à l'agent infectieux, en tirant profit de sa résistance partielle aux protéases.

A ce titre, on peut distinguer :
- les méthodes de Western-blot qui reposent sur la détection immunologique de la PrP^{sc} dans un extrait de tissus, après traitement de l'extrait par une protéase de façon à détruire l'isoforme normale PrP^{c}, la séparation des protéines de l'extrait par électrophorèse, transfert sur une membrane de polymère, et la détection par un anticorps spécifique reconnaissant la PrP (Schaller O. et al., Acta Neuropathol., 1999, 98, 437-443), et
- les tests de type ELISA, qui font également intervenir un traitement des extraits de tissus par une protéase.

Parmi ces différents tests, impliquant un traitement des extraits de tissus par une protéase, on peut citer :
- celui décrit par Serban *et al.* (Neurology, 1990, 40, 100), qui ont développé un test de détection de la PrP^{Sc} qui inclut l'immobilisation des protéines sur une membrane de nitrocellulose, suivie d'une digestion protéasique, d'une dénaturation et d'une immunodétection avec des anticorps monoclonaux,
- celui décrit par Oesch et al. (Biochemistry, 1994, 33, 5926-5931), qui ont proposé, pour quantifier la quantité de PrPSc, un test d'immunofiltration pour la purification de la PrP^{Sc} (ELIFA ou enzyme-linked immunofiltration assay),
- celui décrit par Gratwohl K.U. et al., J. Virol. Methods, 1997, 64, 205-216, qui proposent un dosage de type ELISA. Après traitement des échantillons à la protéinase K et purification de la PrP^{Sc} par centrifugation, celle-ci est adsorbée sur des plaques de microtitration et détectée à l'aide d'anticorps polyclonaux de lapin,
- celui décrit par Safar J. et al., Nature Med., 1998, 10, 1157-1165, qui n'utilise pas la protéinase K et compare l'immunoréactivité de la PrPSc immobilisée sur un support solide selon qu'elle a été préalablement ou non soumise à un traitement dénaturant.

Plus récemment, il a été proposé dans le document WO 01/35104, un test impliquant successivement le traitement d'un échantillon présumé contenir la PrP^{Sc} avec la protéinase K dans des conditions ajustées pour permettre la dégradation complète de la protéine PrP^{C} tout en conservant tout ou partie des motifs octa-peptides répétés de la protéine PrP^{Sc}, la mise en contact consécutive dudit échantillon ainsi traité avec un ligand, notamment un anticorps spécifique des motifs octa-peptides et la détection éventuelle d'un complexe motifs octa-peptide répétés -ligand.

De manière générale, ces différents tests présentent l'inconvénient de manquer de sensibilité et par conséquent d'entraîner des faux-négatifs. De plus, ils sont réalisés en *post-mortem.*

En fait, la détection précoce des prions dans le sang ou l'urine nécessiterait un test 10 à 100 fois plus sensible que ceux actuellement existants. De plus, la spécificité vis-à-vis de l'isoforme anormale, PrP^{Sc}, est une condition indispensable pour la mise au point d'un test performant.

En conséquence, il demeure un besoin pour de nouvelles méthodes de détection d'encéphalopathies, en particulier des méthodes pouvant être appliquées chez l'être vivant, qui soient rapides et qui permettent la détection de la pathologie à un stade très précoce.

La présente invention a précisément pour objet de proposer une nouvelle méthode de diagnostic pour les encéphalopathies qui permet d'établir un diagnostic précoce de la maladie, c'est-à-dire *ante-mortem,* chez l'homme ou l'animal, et qui est non invasive.

Selon un de ses aspects, la présente invention vise à titre principal un procédé utile pour amplifier sélectivement l'oligomérisation et/ou augmenter le taux infectieux d'au moins une isoforme PrP^{Sc} de la protéine du prion cellulaire PrP, le cas échéant en mélange avec l'isoforme PrP^{C}, caractérisé en ce qu'il comprend la mise en présence de ladite isoforme PrP^{Sc}, dans des conditions propices à l'amplification, avec une quantité efficace d'au moins un composé de formule générale I 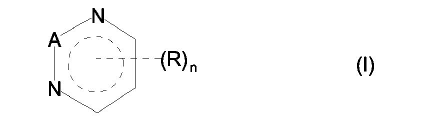 dans laquelle A, R et n sont tels que définis ci-après.

Selon un autre de ses aspects, la présente invention concerne un procédé utile pour détecter la présence ou le risque de survenue d'une encéphalopathie chez un sujet animal ou humain, ledit procédé comprenant au moins :
- la mise en présence d'un échantillon biologique dudit sujet avec une quantité efficace d'au moins un composé de formule générale I tel que défini ci-dessus dans des conditions propices à l'amplification de l'oligomérisation d'isoforme(s) PrP^{Sc} éventuellement présente(s) dans ledit échantillon, et
- la détection de la présence éventuelle de ladite isoforme PrP^{sc}, indicatrice de la présence ou du risque de développement d'une encéphalopathie chez ledit sujet.

Le procédé selon l'invention est particulièrement utile pour la détection d'une encéphalopathie subaiguë spongiforme transmissible (ESST) et notamment d'une maladie de Creutzfeldt-Jakob chez l'homme, la tremblante chez le mouton et la chèvre et l'encéphalopathie spongiforme bovine.

Selon un mode de réalisation particulier, le composé de formule générale I peut être mis en oeuvre sous une forme associée à un marqueur propice à une détection luminescente ou par affmité.

Selon un autre mode de réalisation particulier de l'invention, l'étape de détection implique une opération de détection immunologique, notamment par utilisation d'anticorps spécifiques.

Selon cette seconde variante, cette détection comprend au moins le traitement de tout ou partie dudit échantillon par une protéase, telle que par exemple la protéase K, de façon à détruire l'isoforme normale de la PrP, la séparation des protéines et la détection de l'isoforme PrP^{Sc} éventuellement présente, notamment par un anticorps spécifique. L'étape de destruction de l'isoforme normale de la PrP est généralement réalisée postérieurement à l'étape d'amplification de l'oligomérisation de l'isoforme PrP^{Sc}.

Un mélange d'anticorps SAF60 et SAF69, spécifique des séquences 157-161 de la PrP humaine et SAF70, spécifique de la séquence 156-162 de la PrP humaine, convient notamment à la détection.

De même, le traitement avec la protéinase K peut être réalisé dans les conditions décrites dans le document WO 01/35104 qui conduisent à la dégradation complète de la protéine PrP^{C} tout en conservant tout ou partie des motifs octa-peptides répétés de la protéine PrP^{Sc}. La détection peut être réalisée dans ce cas par la mise en contact consécutive dudit échantillon ainsi traité, avec un ligand notamment des motifs octa-peptides pour détecter l'éventuelle présence de complexe(s) motifs octa-peptide répétés ―ligand.

Selon encore un autre de ces aspects, la présente invention concerne un kit pour l'amplification sélective de l'oligomérisation d'au moins une isoforme PrP^{Sc} ainsi qu'un kit utile pour le diagnostic des ESST, comprenant à titre de réactif au moins un composé de formule générale I.

De manière inattendue, les inventeurs ont constaté que les composés de formule générale I s'avèrent particulièrement efficaces pour amplifier sélectivement l'oligomérisation des isoformes PrP^{Sc} de la protéine Prion cellulaire notamment en présence de la forme non pathogène de celle-ci. Ainsi, les composés de formule générale I ont la particularité d'augmenter la présence de formes oligomériques de PrP^{Sc} sans augmenter celles de l'isoforme normale PrP^{c}. De toute évidence, les composés conformes à l'invention agissent spécifiquement sur les molécules de PrP^{Sc}.

Cette capacité est particulièrement avantageuse dans la mesure où elle permet de diagnostiquer à un stade précoce le risque de survenue d'une encéphalopathie.

En amplifiant sélectivement dans un échantillon biologique des isoformes PrP^{Sc} non détectables en tant que telles avec des méthodes de diagnostic conventionnelles, on rend désormais possible la détection à un stage significativement plus précoce.

Pour des raisons évidentes, un tel diagnostic précoce ne peut être que bénéfique sur les plans curatif et économique et constituer un besoin majeur en santé publique et sur le plan de la sécurité sanitaire.

Ainsi, l'invention rend possible l'élaboration d'un test de détection des prions dans un liquide physiologique tel que l'urine et le sang, pour le diagnostic pré-clinique de la tremblante du mouton et de l'encéphalopathie spongiforme bovine, et ainsi la détection des animaux malades qui actuellement peuvent passer dans la chaîne alimentaire.

En outre, les procédés selon l'invention appliqués sur des échantillons de sang apportent une meilleure garantie concernant les transfusions sanguines humaines et permettent l'établissement d'un test de diagnostic précoce des ESST chez l'homme, voire l'optimisation des traitements futurs.

Comme précisé précédemment, l'invention repose sur l'utilisation du composé de formule générale I comme suit : 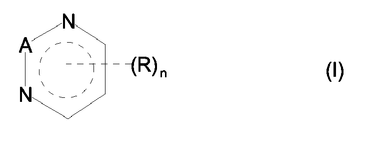 dans laquelle :
- A représente un groupement ―C(H)ₓR₁-, -CO-, C(H)ₓO(R₁), -C(H)ₓS(R₁)-, - CN(R₁)(R₂)- avec x étant égal à 0 ou 1, et R₁ et R₂ représentant, indépendamment l'un de l'autre, un atome d'hydrogène, un radical alkyle en C₁ à C₆ linéaire, ramifié ou cyclique, saturé ou insaturé, le cas échéant substitué,
- le symbole 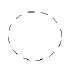 signifie que le cycle pyrimidyle peut être saturé, insaturé ou aromatique,
- R représente un radical choisi parmi :
   - un atome d'hydrogène,
   - un groupement OR'1, N(R'1)(R'2), SR'1 avec R'1 et R'2 représentant, indépendamment l'un de l'autre, un groupement tel que défini précédemment pour R₁ et/ou R₂,
   - un radical hydrocarboné en C₁ à C₁₀, linéaire, ramifié ou cyclique, saturé ou insaturé, et dont la chaîne hydrocarbonée peut être le cas échéant interrompue par un ou plusieurs hétéroatomes choisis parmi les atomes de S, O et N, et le cas échéant substituée,
   - n est égal à zéro ou est un entier variant de 1 à 3,
- et ses sels, solvates, isomères ou dérivés.

En ce qui concerne ses sels, il peut s'agir de tous les sels inorganiques ou organiques dans la mesure où ils s'avèrent compatibles avec la mise en oeuvre de l'invention.

En ce qui concerne les dérivés, il s'agit plus particulièrement au sens de l'invention des composés de formule générale I incorporant au niveau de leur structure au moins une entité, appartenant ou non à un système de marquage, capable de les doter d'un mode de détection par exemple luminescent, à l'image par exemple d'un signal visible en infrarouge ou par fluorescence, comme notamment l'Europium ou par affinité tel qu'un marquage à la biotine dans le cas d'une reconnaissance streptavidine biotine.

L'entité, généralement chimique, destinée à procurer au composé de formule générale I un mode de marquage pour une détection, peut être fixée par des méthodes conventionnelles au niveau de la molécule de formule générale I.

Bien entendu, sa localisation est choisie de manière à ne pas affecter la réactivité attendue du dérivé de formule générale I ainsi obtenu.

Plus particulièrement, le cycle pyrimidyle est un cycle aromatique.

Selon un mode de réalisation particulier, n est égal à 1.

En ce qui concerne A, il s'agit plus particulièrement d'un motif -CS(R₁)- avec R₁ représentant un radical alkyle tel que défini précédemment.

R représente de préférence un radical hétérocyclique en C₅ à C₆ insaturé ou aromatique et incorporant un ou deux hétéroatomes choisis parmi les atomes de S, N et O.

Il s'agit plus particulièrement d'un radical thiényle le cas échéant substitué.

A titre de substituant possible, on peut plus particulièrement citer les atomes d'halogène notamment choisis parmi les atomes de fluor, brome, iode et chlore, et plus particulièrement le brome et les radicaux OH, OR', NR'R", SR', R' avec R' et R" représentant, indépendamment l'un de l'autre, un radical alkyle linéaire ou ramifié en C₁ à C₁₀.

A titre représentatif et non limitatif des composés de formule générale I, on peut plus particulièrement citer le 4-(5-Bromo-2-thiényl)-2-(méthylthio)pyrimidine de formule comme suit : 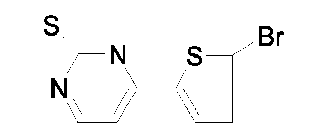 et ses sels ou dérivés.

Compte tenu de leur réactivité vis-à-vis des isoformes PrP^{Sc}, les composés de formule générale I possèdent manifestement la capacité de se lier à au moins un acide aminé.

Sans vouloir se lier à une théorie quelconque, il semblerait que les composés conformes à la présente invention s'avèrent capables de se fixer à au moins l'une des deux régions suivantes de la partie C-terminale de la protéine PrP :
- une région située au niveau du pont disulfure (résidu Cys 179 et Cys 214) qui relie les hélices H2 et H3,
- une région située au niveau de l'hélice H1 (comprenant les résidus 144-154), localisée à l'interface du dimère de PrP dans la structure cristalline.

L'amplification est réalisée généralement par incubation des cellules présumées infectées ou non, avec une quantité efficace en composé(s) de formule générale I. Cette incubation est réalisée dans des conditions propres à l'amplification, généralement à 37 °C, un pH physiologique, pendant une durée suffisante qui peut s'étendre jusqu'à 3 jours environ.

Au sens de la présente invention, "quantité efficace" signifie au moins la quantité minimale et suffisante pour observer l'amplification de l'oligomérisation d'au moins une isoforme PrP^{Sc}, éventuellement présente dans l'échantillon biologique faisant l'objet de l'analyse.

Pour des raisons évidentes, cette quantité est susceptible de varier significativement selon la sensibilité recherchée au niveau de la méthode de diagnostic et/ou la quantité initialement présente en isoforme(s) PrP^{Sc}.

Par exemple, cette concentration peut varier de 0,1 à 20 µM.

Dans le cas d'un mode de détection immunologique des isoformes PrP^{Sc}, celui-ci peut être réalisé par des méthodes conventionnelles de *screening* telles que hybridation ou réaction anticorps/antigènes. Un tel traitement nécessite au préalable la lyse cellulaire en utilisant des méthodes physiques, mécaniques ou chimiques conventionnelles.

Selon une variante de l'invention, les lysats cellulaires ayant subi le procédé d'amplification selon l'invention, peuvent être, au préalable à la détection des isoformes PrP^{Sc}, soumis à une digestion partielle par une protéase et notamment la protéase K de manière à éliminer les molécules de PrP^{C} et donc ne conserver que les isoformes PrP^{Sc} dans l'échantillon biologique. La présence de ces isoformes PrP^{Sc} peut être ensuite détectée par simple analyse conventionnelle, notamment de type immunoblot.

Par exemple, ledit traitement à la PK peut être réalisé de la manière suivante : 1 µg de PK pour 50 ou 25 µg de protéines à digérer pendant 1h à 37 °C pour un total de 200 µg de protéines dans un volume final de 400 µl.

Selon un mode de réalisation particulier de l'invention, ce traitement avec la protéase K peut être également réalisé dans les conditions décrites dans le document WO 01/35104 dont le contenu est incorporé par référence à la présente demande. Ce traitement vise à dégrader complètement l'isoforme normale PrP^{C} tout en conservant tout ou partie des motifs octa-peptides répétés de la PrP^{Sc}, et ce quelque soit la souche d'agent transmissible non conventionnel ATNC.

Il y a lieu de noter qu'un certain nombre de paramètres sont étroitement liés entre eux : la concentration en protéinase K dépend directement de la durée de traitement (temps d'incubation) de l'échantillon : On peut ainsi considérer qu'à 37 °C par exemple, une incubation de 10 minutes avec une PK à un concentration comprise en 30 et 200 µg/ml d'homogénat à 10 % est équivalente à une incubation de 30 minutes avec une PK à une concentration comprise entre 10 µg/ml et 70 µg/ml d'homogénat à 10 %. Par exemple, une incubation pendant 30 minutes avec une PK à 25 µg/ml est équivalente à une incubation de 10 minutes avec une PK à une concentration de 75 µg/ml.

Dans le cas d'une utilisation d'un dérivé de composé de formule générale I c'est-à-dire doté d'un mode de marquage, la détection peut être réalisée selon les conditions standards préconisées pour le marqueur retenu.

La présente invention a en outre pour objet un kit de diagnostic utile pour la mise en oeuvre d'un procédé conforme à l'invention, caractérisé en ce qu'il comprend à titre de réactif au moins un composé de formule générale I.

Selon un autre de ses aspects, la présente invention concerne un kit utile pour le diagnostic d'une ESST caractérisé en ce qu'il comprend au moins un composé de formule générale I en association avec un ligand de la protéine cellulaire PrP et en particulier d'une isoforme PrP^{C}. Plus particulièrement, il s'agit d'un anticorps monoclonal, ou non, ou d'un mélange d'anticorps monoclonaux.

De tels anticorps sont notamment décrits dans Nishida et al. (J. Virol, 74 :320-325, 2000) et commercialisés par la société SPIBIO. Ils sont également décrits dans le brevet WO 01/35104.

Selon un autre de ses aspects, la présente invention concerne un kit utile pour le diagnostic d'une ESST caractérisé en ce qu'il comprend au moins un composé de formule générale I sous la forme d'un dérivé doté d'un système de marquage. Le marquage peut être propice à une détection luminescente, notamment par fluorescence ou par affinité à l'image d'un couple streptavidine/biotine par exemple.

Les exemples et figures figurant ci-après sont présentés à titre illustratif et non limitatif du domaine de l'invention :
**Figures :**

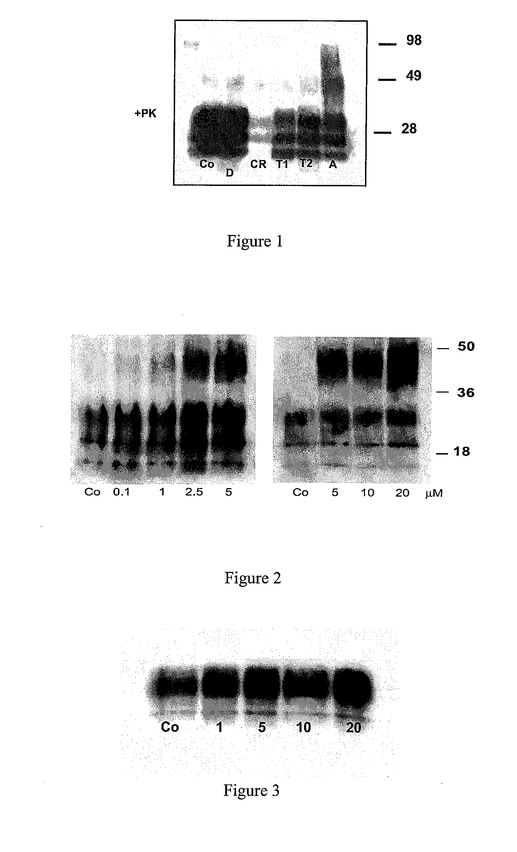

   - Figure 1 : Elle illustre l'activité d'un composé conforme à l'invention sur la PrP^{Sc} de cellules infectées N2a/22L. L'analyse est réalisée par immunoblot après traitement à la protéinase K, (Co = cellules n'ayant subi aucun traitement, D = cellules traitées par la solvant seul (DMSO), CR = Rouge Congo, T1 et T2 sont deux composés témoins inefficaces.
   - Figure 2 : Elle illustre l'activité dose-réponse du composé conforme à l'invention sur la PrP^{Sc} de cellules infectées N2a/22L. L'analyse est réalisée par immunoblot après traitement à la protéinase K. Le composé A a été testé en utilisant une gamme de concentrations de 0,1 à 20 µM, afin d'établir une courbe dose-réponse. (Co = cellules n'ayant subi aucun traitement, les chiffres de 0,1 à 20 correspondent aux concentrations de produit utilisées (en µM).
   - Figure 3 : Elle rend compte de l'effet d'un composé conforme à l'invention sur la PrP^{C}, de cellules non infectées, N2a. L'analyse est réalisée par immunoblot (les chiffres 1 à 10 µM correspondent aux concentrations de produit A utilisé au cours de l'incubation avec les cellules.

Les essais ont été réalisés en utilisant à titre de composé de formule générale I, le composé A, à savoir le 4-(5-Bromo-2-thiényl)-2-(méthylthio)pyrimidine commercialisé par la société MAYBRIDGE sous la référence ACD 30432.

### Exemple 1 : Criblage in vitro sur des cultures cellulaires chroniquement infectées par les prions

Le composé A est testé *in vitro* sur des lignées de neuroblastomes murins chroniquement infectés par la souche de prions 22L (souche de scrapie stabilisée chez la souris) (Nishida N. et al., J. Virol. (2000) 74, 320-325). Cette lignée cellulaire infectée, appelée N2a/22L, produit en grande quantité des molécules de PrP^{Sc} qui sont résistantes à la digestion partielle par la protéinase K, et qui possèdent des propriétés réminiscentes à celles des prions. Les cellules infectées N2a/22L sont capables de provoquer une ESST lorsqu'elles sont inoculées intra-cérébralement aux souris. Les cellules infectées N2a/22L sont incubées avec le composé A, à une concentration de 20 µM pendant une période de 3 jours à 37 °C et 5 % CO₂.

Au terme de 3 jours, les cellules confluentes sont lysées en présence de détergents. Les lysats cellulaires, normalisés au niveau de leur quantité en protéines, sont soumis à une digestion partielle par la protéinase K (PK), afin d'éliminer les molécules PrP^{C} par rapport aux cellules contrôles non traitées. Le produit est testé en duplicat afin d'éliminer les effets non reproductibles.

Dans une seconde étape, le composé A a été retesté en utilisant une gamme de concentrations de 0,1 à 20 µM, afin de voir si un effet dose-réponse pouvait être obtenu. On observe une augmentation du signal d'environ 90 fois à 20 µM sur les formes oligomériques par rapport à un contrôle non traité.

Les figures 1 et 2 rendent compte des résultats obtenus par analyse immunoblot, après traitement de la protéinase K. On note que le composé A augmente de manière remarquable le taux de PrP^{sc} dans les cellules infectées.

Comme les cellules infectées expriment à la fois des molécules de PrP^{c} et de PrP^{sc}, il a été recherché sur laquelle de ces deux isoformes le composé A agissait. En particulier, il a été recherché si les oligomères qui migrent à 49 kDa et 98 kDa (figure 1) étaient des oligomères de PrP^{c} devenus plus résistants à la protéinase K consécutivement à leur interaction avec le composé A. Pour cela, des cellules non infectées qui expriment seulement l'isoforme PrP^{c} ont été incubées en présence du composé A. Les résultats présentés en figure 3 montrent que le composé A n'augmente pas l'oligomérisation de la PrP^{C} dans les cellules.

## Revendications

1. Procédé utile pour amplifier sélectivement l'oligomérisation d'au moins une isoforme PrP^{Sc} de la protéine du prion cellulaire PrP^{C}, **caractérisé en ce qu'**il comprend la mise en présence de ladite isoforme PrP^{Sc}, dans des conditions propices à l'amplification, avec une quantité efficace d'au moins un composé de formule générale I 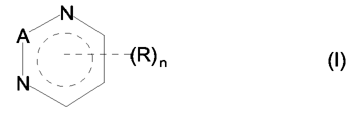 dans laquelle :
- A représente un groupement ―C(H)ₓR₁-, -CO-, -C(H)ₓO(R₁)-, - C(H)ₓS(R₁)-, -CN(R₁)(R₂)- avec x étant égal à 0 ou 1, R₁ et R₂ représentant indépendamment l'un de l'autre un atome d'hydrogène, un radical alkyle en C₁ à C₆ linéaire, ramifié ou cyclique, saturé ou insaturé, le cas échéant substitué,
- le symbole signifie que le cycle pyrimidyle peut être saturé, insaturé ou aromatique,
- R représente un radical choisi parmi :
- un atome d'hydrogène,
- un groupement OR'₁, N(R'₁)(R'₂), SR'₁ avec R'₁ et R'₂ représentant, indépendamment l'un de l'autre, un groupement tel que défini précédemment pour R₁ et/ou R₂,
- un radical hydrocarboné en C₁ à C₁₀, linéaire, ramifié ou cyclique, saturé ou insaturé, et dont la chaîne hydrocarbonée peut être le cas échéant interrompue par un ou plusieurs hétéroatomes choisis parmi les atomes de S, O et N, et le cas échéant substituée,
- n est égal à zéro ou est un entier variant de 1 à 3, et
- ses sels, solvates, isomères ou dérivés.

2. Procédé selon la revendication 1, **caractérisé en ce que** l'amplification est effectuée en présence de l'isoforme PrP^{c}.

3. Procédé selon l'une quelconque des revendications précédentes, **caractérisé en ce que** le cycle pyrimidyle du composé de formule générale I est un cycle aromatique.

4. Procédé selon l'une quelconque des revendications précédentes, **caractérisé en ce qu'**en formule générale I, n est égal à 1.

5. Procédé selon l'une quelconque des revendications précédentes, **caractérisé en ce que** A figure un motif -CS((R₁)- avec R₁ représentant un radical alkyle tel que défini en revendication 1.

6. Procédé selon l'une quelconque des revendications précédentes, **caractérisé en ce qu'**en formule générale I, R représente un radical hétérocyclique en C₅ à C₆, insaturé ou aromatique, et incorporant un ou deux hétéroatomes choisis parmi les atomes de S, N et O.

7. Procédé selon l'une quelconque des revendications précédentes, **caractérisé en ce que** R représente un radical thiényle.

8. Procédé selon l'une quelconque des revendications précédentes, **caractérisé en ce que** ledit composé de formule générale I est capable de se lier à au moins un acide aminé.

9. Procédé selon l'une quelconque des revendications précédentes, **caractérisé en ce que** le composé est le 4-(5-Bromo-2-thiényl)-2-(méthylthio)pyrimidine ou l'un de ses sels ou dérivés.

10. Procédé selon l'une quelconque des revendications précédentes, **caractérisé en ce que** ledit composé de formule générale est sous la forme d'un de ses dérivés incorporant au moins une entité capable de le doter d'un mode de détection.

11. Procédé selon la revendication précédente, **caractérisé en ce que** ladite entité est un marqueur propice à une détection luminescente ou à une détection par affinité.

12. Procédé utile pour détecter la présence ou le risque de survenue d'une encéphalopathie chez un sujet, ledit procédé comprenant au moins :
- la mise en présence d'un échantillon biologique dudit sujet avec une quantité efficace d'au moins un composé de formule générale I tel que défini en revendication 1 et 3 à 11 dans des conditions propices à l'amplification d'isoforme(s) PrP^{Sc} éventuellement présente(s), et
- la détection de la présence éventuelle de ladite isoforme PrP^{Sc}, indicatrice de la présence ou du risque de développement d'une encéphalopathie chez ledit sujet.

13. Procédé selon la revendication 12, utile pour la détection d'une encéphalopathie subaiguë spongiforme transmissible (ESST).

14. Procédé selon la revendication 12 ou 13, utile pour la détection d'une maladie de Creutzfeldt-Jakob chez l'homme, la tremblante chez le mouton et la chèvre et l'encéphalopathie spongiforme bovine.

15. Procédé selon l'une quelconque des revendications 12 à 14, **caractérisé en ce que** le composé de formule générale I est utilisé sous la forme d'un dérivé doté d'un système de marquage.

16. Procédé selon la revendication précédente, **caractérisée en ce que** le dérivé incorpore un marqueur propice à une détection luminescente ou à une détection par affinité.

17. Procédé selon l'une quelconque des revendications 12 à 16, **caractérisé en ce qu'**on réalise en outre le traitement de tout ou partie dudit échantillon traité par une protéase de façon à détruire l'isoforme normale de la PrP^{C}, que l'on sépare les protéines et l'on détecte l'isoforme PrP^{Sc} éventuellement présente par un anticorps spécifique.

18. Procédé selon la revendication précédente, **caractérisé en ce que** la protéase utilisée est la protéase K.

19. Procédé selon la revendication précédente, **caractérisé en ce que** le traitement avec la protéase K est réalisé dans des conditions propices à la dégradation complète de la protéine PrP^{C} tout en conservant tout ou partie de la protéine PrP^{Sc} et **en ce que** l'on réalise la détection consécutive d'isoforme(s) PrP^{Sc}.

20. Kit utile pour l'amplification sélective de l'oligomérisation d'au moins une isoforme PrP^{Sc} de la protéine du prion cellulaire PrP^{C}, **caractérisé en ce qu'**il comprend à titre de réactif au moins un composé de formule générale I tel que défini dans les revendications 1 et 3 à 11.

21. Kit utile pour le diagnostic des encéphalopathies, et en particulier des encéphalopathies subaiguës spongiformes transmissibles (ESST) animales ou humaines, comprenant à titre de réactif au moins un composé de formule générale I tel que défini en revendications 1 et 3 à 11 sous la forme d'un dérivé doté d'un système de marquage.

22. Kit utile pour le diagnostic des encéphalopathies, et en particulier des encéphalopathies subaiguës spongiformes transmissibles (ESST) animales ou humaines, comprenant à titre de réactif au moins un composé de formule générale I tel que défini en revendications 1 et 3 à 11 en association avec un ligand se liant spécifiquement à une isoforme PrP.

23. Kit selon la revendication 21 ou 22, **caractérisé en ce qu'**il comprend en outre un anticorps ou mélange d'anticorps reconnaissant une isoforme PrP^{sc}.
